# FASCICULE DE BREVET EUROPEEN

(11) **EP 4 255 559 B1**
(45) Date de publication et mention de la délivrance du brevet: **08.04.2026**
(21) Numéro de dépôt: 21810645.8
(22) Date de dépôt: 29.11.2021
(51) Int. Cl.: A61N 5/06, A45D 29/00, A45D 29/22, A45D 29/14

(54) **SYSTÈME ET PROCÉDÉ POUR LE TRAITEMENT DES ONGLES**
SYSTEM UND VERFAHREN ZUR BEHANDLUNG DER NÄGEL
SYSTEM AND METHOD FOR TREATING THE NAILS

(30) Priorité: 03.12.2020 FR 2012647
(43) Date de publication de la demande: 11.10.2023
(73) Titulaire: Eurofeedback, 91090 Lisses (FR)
(72) Inventeur: SAFRAOUI, Georges, 91140 VILLEJUST (FR)
(74) Mandataire: Ipsilon
(86) Numéro de dépôt international: PCT/EP2021/083366
(87) Numéro de publication internationale: WO 2022/117503

(56) Documents cités:
- US-A- 6 090 788
- US-A1- 2009 048 590
- US-A1- 2009 143 842
- US-A1- 2012 109 265
- US-A1- 2013 211 481
- US-A1- 2014 194 955
- US-A1- 2014 288 621

## Description

### Domaine technique

La présente invention concerne le traitement des ongles des mains ou des pieds.

### Technique antérieure

De nombreuses personnes, surtout âgées, soufrent d'affections diverses des ongles, notamment d'onychomycoses.

Les traitements classiques qui reposent sur l'utilisation de vernis antifongiques, sont relativement contraignants car devant s'effectuer sur une longue durée, et présentent en outre les inconvénients inhérents à l'utilisation de composés présentant une certaine toxicité.

La demande US 2013/211481 décrit un revêtement de type pansement liquide durcissant, infusé avec un agent antifongique, pouvant comporter un colorant qui change de couleur à une certaine température lorsqu'exposé à la source de lumière.

Il est connu d'utiliser la lumière, et en particulier la lumière pulsée, pour tenter d'améliorer l'aspect esthétique de l'ongle ou son état pathologique.

Le brevet US 6 090 788 divulgue une méthode de photothérapie pour traiter les infections fongiques de l'ongle, dans laquelle on associe à l'agent pathogène un pigment absorbant la lumière avant son irradiation par un faisceau lumineux.

Les demandes US 2009/143842, US 2014/288621, US 2014/194955 et US 2012/109265 divulguent d'autres dispositifs lumineux de traitement des infections fongiques ou bactériennes des ongles.

D'autres essais de traitements par la lumière pulsée ont été réalisés pour tenter d'améliorer l'aspect esthétique de l'ongle ou son état pathologique. La préparation des ongles pour le traitement est alors limitée à un ponçage éventuel de la surface extérieure de l'ongle afin d'enlever les surépaisseurs. L'ongle est ensuite exposé pendant quelques minutes à une séquence de flashs répétés, de fluence suffisante afin d'élever la température de l'ongle.

Dans le cas d'une source à lampe flash polychromatique (IPL), les flashs de lumière divergente sont envoyés d'une distance suffisante pour ne pas brûler l'environnement cutané de l'ongle, et la température à la surface de l'ongle se situe au-dessous des 50°C (45°C étant considéré comme la limite de la zone d'inconfort). En général, 4 à 6 séances espacées de 3 à 4 semaines sont effectuées.

Dans le cas de l'utilisation d'une source de lumière laser, c'est la solution YAG à 1054nm qui est utilisée. La zone traitée par le laser est limitée à un spot de quelques mm², ce qui nécessite de nombreux tirs pour traiter toute la surface de l'ongle. De plus, la fluence est relativement importante, de l'ordre de 70J/cm², du fait de la faible absorption des tissus. Comme pour le traitement par lampe flash polychromatique, la température ne dépasse pas les 50°C à la surface de l'ongle afin de ne pas générer de brûlure, ou d'inconfort.

L'efficacité de tels traitements n'est pas démontrée, du fait de la faible température atteinte. De plus, la durée du traitement par laser est relativement longue, de l'ordre de plusieurs minutes par ongle, ce qui rend le traitement fastidieux.

Ainsi, les traitements par la lumière des ongles, tels qu'effectués aujourd'hui, restent relativement peu efficaces, comme en témoignent différentes études telle que celle objet de l'article « The effectiveness of lasers in the treatment of onychomychosis : a systematic review » Bristow Journal of Foot and Ankle Research 2014, 7:34.

### Exposé de l'invention

Il demeure par conséquent un besoin pour améliorer l'efficacité des traitements par la lumière des ongles visant à améliorer leur aspect esthétique et/ou à traiter des pathologies telles que les onychomycoses.

L'invention concerne un système et un procédé tels que définis par les revendications jointes.
Les exemples ou les modes de réalisation non couverts par la portée des revendications jointes, notamment les procédés de traitement, doivent être considérés uniquement comme des exemples permettant de comprendre l'invention.

### Résumé de l'invention

L'invention vise à répondre à ce besoin, et elle y parvient selon un premier de ses aspects grâce à un procédé de préparation d'un ongle en vue de lui faire subir un traitement par impulsions lumineuses selon la revendication 14.

Par « revêtement absorbant » il faut comprendre que le revêtement absorbe une part significative de la lumière émise par le dispositif d'émission d'impulsions lumineuses, et améliore la transformation de la lumière en chaleur comparativement à l'ongle nu. Avantageusement, la réflectivité R du revêtement est inférieure à 50%, mieux à 40%, 30% ou 20%, et/ou sa transmissivité T est inférieure à 50%, mieux à 40%, 30% ou 20%.

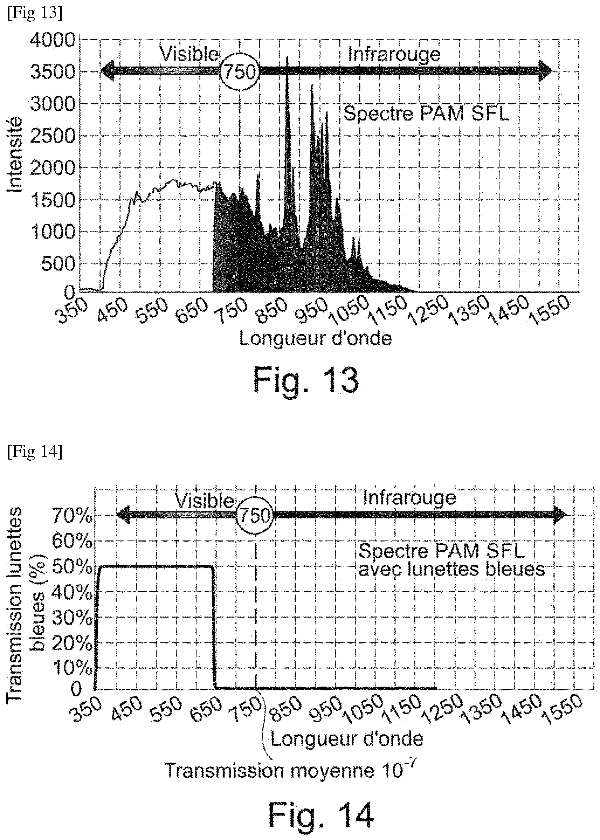

De préférence, on a une réflectivité et/ou une transmissivité du revêtement selon l'invention inférieures à 50%, mieux à 40%, 30% ou 20%, vis-à-vis d'une lumière dite à spectre SFL (Super Filtered Light) pouvant être utilisée pour le traitement, dont la distribution spectrale de l'intensité est représentée à la figure 13. On voit sur cette figure que l'intensité substantielle est émise après 675nm environ (en partie pleine sur la figure), le spectre d'émission s'étendant jusqu'autour de 1200nm. On a représenté en trait léger le spectre émis en dessous de 675nm par la lampe, un filtre interne au dispositif bloquant la lumière sortante pour donner le spectre d'émission correspondant à la partie pleine. Le spectre SFL est par exemple émis par une machine de marque FLUENCE, ARIANE ou ANTHELIA de la société EUROFEEDBACK.

La réflectivité R correspond à la portion d'énergie lumineuse réfléchie Er à la surface du revêtement, rapportée à l'énergie incidente Eo, compte-tenu du spectre d'émission de celle-ci. Elle varie de 0 pour les corps noirs à 1 pour un miroir parfait.

La transmissivité T est définie comme étant le rapport de l'énergie de la lumière transmise Et rapportée à l'énergie de la lumière incidente Eo. Pour une réflectivité R donnée, moins un corps est transmissif, plus il est absorbant, avec 1=R+T+A, où A est l'absorptivité.

Selon l'invention, l'absorptivité A du revêtement absorbant (exprimée en %) est supérieure ou égale à 50%, mieux à 60%, encore mieux à 70%, voire à 80% ou 90%, idéalement plus de 95% ou 99%.

La transmissivité T peut se mesurer avec un Joule-mètre de précision, en mesurant le rapport entre l'énergie de la lumière incidente et celle de la lumière sortant à travers le revêtement.

Pour un exemple de revêtement en polyimide (Kapton) noir, on a par exemple une transmissivité T inférieure à 20%, par exemple de l'ordre de 15%, et pour un autre exemple de revêtement, en PTFE noir, on a une transmissivité inférieure à 10%, par exemple de l'ordre de 5%. Dans le premier cas, pour une énergie entrante de 30J, on a environ 4J en sortie, et dans le deuxième cas, pour une énergie entrante de 30J on a environ 2J en sortie.

Pour comparaison, la réflectivité naturelle des ongles sains est de l'ordre de 50% (pour une longueur d'onde de 800nm) pour une épaisseur d'ongle de 1mm ; les ongles nus absorbent par exemple seulement 8% environ de la lumière incidente, de sorte que l'énergie absorbée par les ongles est limitée à 4% environ de l'énergie incidente.

Le revêtement disposé sur l'ongle permet d'absorber artificiellement la lumière émise par le dispositif et sa température peut augmenter très rapidement et se propager sous la forme d'une « vague thermique » à travers l'ongle jusqu'au-tissu sous-jacent. L'élévation de température qui en résulte permet de détruire efficacement les mycoses ou autres agents pathogènes qui affectent l'ongle, sans toutefois causer de brûlure du fait de sa courte durée, De plus, comme le revêtement opaque ne s'étend que sur l'ongle, l'environnement cutané autour de l'ongle ne subit pas la même élévation de température, et peut être préservé. L'invention permet d'améliorer l'efficacité du traitement des ongles à puissance et fluence moindres.

L'invention permet un traitement simultané de plusieurs ongles en même temps, si on le souhaite, car la plus faible puissance lumineuse requise peut permettre d'étaler davantage la lumière.

L'invention permet un traitement sélectif et sécurisé, car il n'échauffe pas la peau outre mesure autour de l'ongle, et permet de travailler sur des peaux noires, le cas échéant, en protégeant la peau, comme expliqué plus loin.

La température à la surface du revêtement est par exemple comprise à l'issue de la rafale d'impulsions reçue entre 180 et 250°C, pendant une fraction de seconde. Par exemple, la température à la surface du revêtement est de l'ordre de 200°C ou plus pendant 0,2s environ après la fin de la rafale de flashs. Malgré cette température élevée, qui redescend rapidement à la fin du dernier flash, l'utilisateur ne ressent qu'une sensation d'inconfort ou une légère sensation de brûlure, mais qui disparait rapidement. La température sous l'ongle atteint par exemple 70°C pendant environ 1s, car l'ongle est un isolant thermique et a de plus une constante de temps thermique TRT (temps de relaxation thermique) de plus d'une seconde. Ainsi, toutes les moisissures ayant colonisé la matière de l'ongle et se nourrissant de cette dernière sont par exemple soumises à des températures allant de 100°C à 200°C pendant une fraction de seconde, mais suffisante pour les détruire.

Les puissances peuvent être choisies afin de ne traiter que la partie phanère de l'ongle, et non les tissus sous-jacents, à des températures élevées, par exemple de l'ordre de 180°C en surface et 100°C au centre de l'ongle, ce qui permet de détruire efficacement et relativement rapidement les champignons tout en limitant l'inconfort de l'utilisateur. Ainsi, la partie phanère peut être traitée à une température supérieure à 65°C tandis que la partie tissu sous-jacente, vascularisée et innervée, n'est pas soumise à une température supérieure à 65°C.

Le revêtement permet de transformer en chaleur une large fraction de l'énergie de la lumière incidence, par exemple plus de 20 fois l'énergie qui serait absorbée par l'ongle en l'absence de revêtement.

De préférence, on applique à la périphérie de l'ongle un revêtement réfléchissant, notamment blanc, de préférence un revêtement dispersable à l'eau. Un tel revêtement permet de réfléchir la lumière et ainsi de limiter encore davantage l'élévation de température de la peau à la périphérie de l'ongle. Cela permet de soumettre le revêtement absorbant disposé sur l'ongle à une rafale d'impulsions lumineuses sans craindre de brûler la peau s'étendant à proximité immédiate de l'ongle.

De préférence, le revêtement opaque est constitué par un film préformé, de préférence un film résistant à une température supérieure ou égale à 60°C, mieux à 80°C, encore mieux à 100°C, à 150°C ou à 200°C, encore mieux à 250°C, mieux à 300°C, de préférence en polyimide ou en PTFE. On choisira la température à laquelle le revêtement résiste en fonction de la puissance surfacique sur le revêtement ; une résistance à la température plus élevée permet d'atteindre une température plus haute, ce qui tend à améliorer l'efficacité du traitement et à le raccourcir.

Par « résistant à une température T », il faut comprendre que le revêtement ne se décompose pas sous l'effet de la chaleur à cette température T, notamment ne brûle pas, et qu'il est vendu comme étant utilisable à cette température.

Par « préformé » il faut comprendre que le film est déjà cohésif avant d'être appliqué sur l'ongle. Le revêtement opaque peut être un film, de préférence autoadhésif, résistant à la température, de préférence résistant à une température d'au moins 60°C, mieux d'au moins 80°C, encore mieux d'au moins 100°C, préférentiellement d'au moins 150°C, et très préférentiellement d'au moins 200°C, mieux d'au moins 250°C, encore mieux d'au moins 300°C, par exemple un film de Kapton adhésif noir. Ce film peut être présent initialement sur une feuille de support antiadhérente. L'épaisseur du film est de préférence supérieure ou égale à 50 microns, mieux à 100 microns. L'épaisseur du film est par exemple inférieure à 1mm, étant de préférence comprise entre 100 et 150 microns. De préférence, on utilise des matières plastiques de classe H ou au-delà (selon la norme IEC 60085 sur la classe thermique des isolants) pouvant tenir par exemple 327°C pour le PTFE et 400°C pour le Kapton. On peut également utiliser certains polyamides résistant à la chaleur, entre autres matériaux utilisables.

Le caractère absorbant du revêtement peut être dû à la présence de pigments ou colorants noirs ou sombres, par exemple des pigments minéraux. Le revêtement peut comporter du graphite ou des nanotubes de carbone, l'invention n'étant pas limitée à un revêtement particulier.

De préférence, le film est prédécoupé sensiblement au format de l'ongle à traiter, ou au format d'une partie de l'ongle, formant ainsi un patch en une ou plusieurs parties à appliquer sur l'ongle. On peut notamment positionner sur l'ongle au moins deux parties prédécoupées, chacune ayant un bord arrondi que l'on peut ajuster au moment du collage sur l'ongle pour épouser au mieux la forme du bord de celui-ci, les deux patchs se recouvrant l'un l'autre sur l'ongle. Ce chevauchement, par exemple au milieu de l'ongle, permet de bien couvrir les bords des ongles. Les patchs peuvent être découpés manuellement ou à l'aide de tout instrument ou appareil adapté.

Bien que l'utilisation d'un film préformé constitue un moyen particulièrement rapide et efficace pour préparer l'ongle, on peut encore sans sortir du cadre de l'invention appliquer le revêtement à l'état fluide sur l'ongle, à l'aide d'un applicateur. Par exemple, le revêtement est formé par séchage d'une composition appliquée à l'état fluide sur l'ongle, par exemple un vernis absorbant résistant à la température et comportant une dispersion d'un pigment opaque dans un liant, le pigment étant de préférence de couleur sombre, notamment noire, par exemple un oxyde métallique, notamment de fer, ou du carbone. Ce vernis peut être filmogène, afin de pouvoir être retiré par pelage. Il peut encore être dispersable à l'eau, afin de faciliter son enlèvement. Le liant du vernis est de préférence choisi pour présenter la résistance à la température nécessaire, notamment résister à une température supérieure ou égale à 60°C, mieux à 80°C, encore mieux à 100°C, mieux à 150°C, préférentiellement à 200°C, mieux à 250°C, encore mieux à 250°C.

On peut encore appliquer sur l'ongle un revêtement opaque sous la forme d'une encre ou d'une poudre, par exemple à l'aide d'un crayon feutre à encre absorbante, de préférence de couleur sombre, mieux noire, par exemple une encre indélébile noire. Un inconvénient de l'utilisation d'une telle encre peut alors être la nécessité d'utiliser un solvant pour son enlèvement, de sorte que l'utilisation d'un revêtement sous forme de film préformé est préférée.

Le revêtement absorbant peut le cas échéant être déposé sur un vernis préalablement appliqué, afin d'être plus facilement retirable par pelage ou nettoyage. Cet autre vernis peut être transparent. Cet autre vernis est résistant à la température, et supporte de préférence une température de 60°C, mieux de 80°C, encore mieux de 100°C, mieux de 150°C, préférentiellement de 200°C et plus préférentiellement de 250°C.

De préférence, le procédé de préparation de l'ongle comporte l'étape consistant à abraser la surface de l'ongle à traiter préalablement à l'application du revêtement absorbant. Cela permet de lisser la surface de l'ongle et de diminuer l'épaisseur de l'ongle. Le lissage permet d'améliorer la qualité du contact thermique du revêtement avec l'ongle, notamment lorsque le revêtement est sous forme de film préformé. Le ponçage de l'ongle peut être réalisé de manière à avoir une épaisseur restante d'ongle comprise entre 1 et 2mm, mieux de 1mm environ, sur sensiblement toute la surface de l'ongle, laquelle sera recouverte par le revêtement absorbant.

Notamment dans le cas où la lumière émise est générée par au moins une lampe flash, l'ongle est de préférence positionné à une certaine distance de la fenêtre de sortie de la lumière, en vue d'illuminer de manière relativement uniforme l'ensemble du revêtement présent sur l'ongle. Le procédé peut ainsi comporter l'étape consistant à positionner un support de telle sorte que l'ongle se situe à une distance comprise entre 1 et 6 cm, notamment 2 et 5cm, de la fenêtre de sortie de la lumière. De préférence, dans le cas d'une source laser, on peut positionner la fenêtre de sortie de la lumière et choisir la divergence du faisceau de telle sorte que chaque impulsion laser irradie toute la surface du revêtement absorbant présent sur l'ongle. La fenêtre de sortie peut être écartée d'une distance de 0 à 20cm, par exemple, de la surface du revêtement.

De préférence, on protège au mieux toute la surface de peau exposée à la lumière, et l'on peut ainsi disposer au-dessus de la peau du doigt un écran protecteur, notamment une feuille d'un matériau souple et réfléchissant, en particulier une feuille d'un papier ou d'un textile blanc. Il peut subsister des zones de peau non protégées de la lumière, mais de préférence non situées dans le voisinage immédiat de l'ongle. Pour les peaux sombres, il est préférable de masquer totalement la peau.

Lorsque seul un ongle est à traiter, le doigt portant cet ongle peut être isolé des autres doigts par un écran protecteur, notamment une feuille d'un matériau souple et réfléchissant, en particulier une feuille d'un papier ou d'un textile blanc.

Lorsque l'on prépare plusieurs ongles en vue de les traiter simultanément, notamment deux ou trois, voire tous ceux du pied, on peut positionner la fenêtre de sortie de manière à pouvoir exposer simultanément ces ongles à la lumière émise.

Des séparateurs interdigitaux peuvent être disposés entre les doigts, le cas échéant.

Une fois le traitement effectué, le revêtement absorbant peut être retiré, par exemple par simple pelage dans le cas d'un film préformé adhésif ou adhérant de façon électrostatique.

L'invention a encore pour objet, selon un autre de ses aspects, un procédé pour améliorer l'aspect d'au moins un ongle, comportant l'étape consistant à préparer l'ongle par la mise en œuvre du procédé selon l'invention tel que défini ci-dessus, et à soumettre l'ongle ainsi revêtu à au moins une impulsion, et mieux au moins une rafale d'impulsions lumineuses.

Chaque impulsion peut être de fluence, mesurée sur le revêtement, comprise entre 0,1 et 10 J/cm², mieux entre 0,5 et 5J/cm², encore mieux comprise entre 0,5 et 2 J/cm², par exemple de l'ordre de 1J/cm². On soumet par exemple le revêtement sur l'ongle à une énergie par cm² comprise entre 5 et 30J pendant la durée totale de la rafale, cette durée étant par exemple comprise entre 1 et 10s, mieux entre 2 et 10s, par exemple entre 3 et 6s.

Pour obtenir 1J/cm² dans le cas d'un IPL générant une lumière polychromatique divergente, la fluence en sortie de la tête de l'IPL peut être de l'ordre de 4 fois plus importante, par exemple de l'ordre de 4J/cm². Du fait de la distance entre la tête de l'IPL et le revêtement et de la divergence du faisceau, la fluence mesurée sur le revêtement est moindre, par exemple environ 4 fois moindre.

La densité de puissance surfacique, mesurée sur le revêtement, est par exemple en moyenne au cours de la rafale comprise entre 0,5 et 10 W/cm², mieux entre 1 et 10 W/cm², encore mieux entre 1 et 5 W/cm². Par exemple, à 3Hz on émet par seconde trois flashs de 4J/cm² chacun à la sortie du dispositif, ce qui correspondant à 12J/cm² par seconde, mais si compte-tenu de l'éloignement de l'ongle ce dernier en reçoit par exemple environ 4 fois moins, soit 3J/cm², on a alors une densité de puissance moyenne surfacique de 3W/cm². Chaque flash de 4J/cm² peut avoir une durée de quelques dizaines de millisecondes.

Il est possible de ne traiter qu'un seul ongle à la fois. On peut encore exposer simultanément au moins deux ongles à l'impulsion lumineuse, comme mentionné ci-dessus.

De préférence, on soumet l'ongle à un nombre d'impulsions lumineuses compris entre 2 et 100, mieux entre 2 et 50, notamment entre 5 et 20.

La fréquence d'émission des impulsions lumineuses peut aller de 1 à 10 Hz, de préférence de 1 à 5Hz, notamment 2 à 4 Hz. Il peut y avoir environ 1/f en s entre chaque flash, la durée d'un flash étant très courte (de l'ordre d'une dizaine de millisecondes). Pour une fréquence relativement rapide, la température de l'ongle n'a pas le temps de diminuer sensiblement entre deux flashs successifs, et ainsi tend à augmenter progressivement au cours des flashs de la rafale, pour devenir maximale à la fin du dernier flash de la rafale.

Après l'émission d'une rafale, on laisse de préférence l'ongle refroidir pendant une durée d'au moins 30s, mieux d'au moins 1mn, encore mieux une durée comprise entre 1 et 5 mn, avant de le soumettre à une nouvelle rafale.

L'ongle peut n'être soumis qu'à deux rafales successives lors d'une cession de traitement. Deux cessions de traitement peuvent être espacées d'au moins une semaine. L'ongle peut être soumis au besoin à un nombre de rafales plus grand.

La lumière peut être émise par au moins une lampe flash polychromatique (IPL) ou par un laser. L'utilisation d'un dispositif de traitement IPL à lampe flash est pratique, car permet de traiter aisément plusieurs ongles simultanément.

Lors du traitement, la température maximale mesurée à la surface du revêtement peut dépasser 60°C, mieux 80°C, encore mieux 100°C, mieux 150°C, mieux 200°C, étant de préférence comprise entre 150°C et 300°C. Une température supérieure à 150°C, mieux à 180°C, encore mieux à 200°C, est atteinte de préférence pendant une durée d'au moins 0,1s, mieux pendant une durée comprise entre 0,1 et 0,5s, encore mieux pendant une durée comprise entre 0,1 et 0,3s. Une température supérieure à 150°C est préférée, car elle permet de raccourcir le traitement tout en présentant une bonne efficacité. Une température plus faible, associée à une durée de traitement plus longue, peut être retenue lorsque la puissance de la source lumineuse est plus faible, tout en veillant à rester en deçà du seuil de brûlure.

L'invention a encore pour objet, selon un autre de ses aspects, un système selon la revendication 1 pour le traitement des ongles, notamment pour la mise en œuvre du procédé de traitement tel que défini ci-dessus, comportant :
- Un dispositif d'émission de lumière pulsée permettant d'exposer au moins un ongle à traiter à au moins une impulsion lumineuse, mieux à au moins une rafale d'impulsions lumineuses,
- au moins un revêtement à absorbant la lumière émise par le dispositif, à appliquer sur le ou les ongles, de préférence un revêtement de réflectivité inférieure à 50% et/ou de transmissivité inférieure à 50%, notamment un revêtement de couleur sombre, ou un applicateur d'un tel revêtement, et dont la température de surface est apte à dépasser 60°C, mieux 80°C, encore mieux 100°C ou 150°C, mieux 200°C, sous l'effet de la lumière émise par le dispositif.

Chaque impulsion lumineuse peut présenter tout ou partie des caractéristiques mentionnées ci-dessus.

Le revêtement absorbant peut présenter tout ou partie des caractéristiques déjà mentionnées ci-dessus. Le revêtement est d'absorptivité supérieure ou égale à 50%. Le système ainsi peut comporter un film opaque absorbant la lumière émise par le dispositif, de préférence de couleur sombre, notamment noire, résistant à une température d'au moins 60°C, mieux d'au moins 80°C, encore mieux d'au moins 100°C ou 150°C, préférentiellement d'au moins 200°C, mieux d'au moins 250°C, encore mieux 300°C, voire 350°C ou 400°C, notamment en PTFE ou en polyimide, en particulier en Kapton.

Ce film est de préférence recouvert sur sa face intérieure à appliquer sur l'ongle d'un adhésif sensible à la pression. En variante, il ne comporte pas d'adhésif et adhère électrostatiquement.

Le film est de préférence prédécoupé pour former un patch à la forme d'un ongle ou d'une partie de l'ongle, ou un ensemble de patchs prédécoupés chacun à la forme d'un ongle ou d'une partie de l'ongle, notamment avec des parties destinées à se chevaucher sur l'ongle. Le film peut être prédécoupé pour former au moins un patch ayant un contour en forme de **D** par exemple.

On peut ainsi avoir sur un support, notamment antiadhésif, un ensemble de patchs notamment autoadhésifs ou adhérant électrostatiquement à l'ongle, résistant à la chaleur, chacun en forme de D, regroupés par paires sur le support, deux patchs d'une paire étant destinés à être posés sur un même ongle avec un chevauchement entre eux.

Le cas échéant, le système peut comporter un applicateur pour appliquer le revêtement à l'état fluide sur l'ongle, par exemple un applicateur de type pinceau ou pointe feutre.

Le système peut comporter un applicateur d'un revêtement réfléchissant, notamment blanc, pour former un écran réfléchissant sur la peau à la périphérie de l'ongle.

Le système peut comporter un dispositif pour abraser mécaniquement la surface de l'ongle, notamment une ponceuse à ongle ou de pédicure.

Le dispositif peut présenter des caractéristiques d'émission telles que la lumière permette d'élever la température du revêtement absorbant présent sur l'ongle pendant la rafale et/ou à l'issue de celle-ci à une température supérieure à 60°C, mieux à 80°C, encore mieux à 100°C, préférentiellement à 150°C, mieux à 180°C, encore mieux à 200°C.

De préférence, le dispositif d'émission de lumière pulsée permet d'émettre une rafale d'impulsions lumineuses à une fréquence comprise entre 0,5 et 20Hz, mieux entre 1 et 5 Hz, chaque impulsion présentant une fluence telle, compte-tenu de la distance séparant une fenêtre de sortie du dispositif du revêtement pendant l'utilisation, qu'elle soit comprise entre 0,5 et 5 J/cm² au niveau de la surface du revêtement, mieux 0,5 et 2 J/cm², le nombre d'impulsions étant de préférence compris entre 2 et 20, mieux entre 5 et 20, notamment entre 10 et 20.

De façon avantageuse, le dispositif d'émission émet sensiblement entre des longueurs d'ondes 650nm et 1200nm, avec par exemple un pic autour de 875nm, le système comportant une paire de lunettes de protection ou un masque de protection de l'opérateur, comportant un filtre absorbant la lumière émise par le dispositif d'émission, ce filtre ayant un facteur de transmission inférieur ou égal à 1% au-delà de 650nm environ. De préférence, le filtre absorbant des lunettes est de couleur bleue.

Cela permet à l'opérateur utilisant le dispositif de ne pas être ébloui par la lumière émise, et ainsi de pouvoir ainsi contrôler facilement l'application de la lumière pendant la mise en œuvre du traitement.

Le système comporte avantageusement un support pour maintenir une fenêtre de sortie, par laquelle la lumière sort du dispositif, à une distance prédéfinie d'une surface de réception de l'ongle à traiter. L'ongle peut être positionné sur cette surface de réception, sous la fenêtre de sortie de la lumière.

Cette distance est comprise par exemple entre 1 et 6 cm. La surface du revêtement absorbant peut ainsi se trouver à une distance comprise entre 0 et 5 cm de la fenêtre de sortie, par exemple. La distance peut être choisie en fonction de la divergence de la lumière, pour couvrir entièrement un ongle, voire deux, voire plus. Dans le cas d'un laser, dont la divergence est réglable, on peut avoir une fenêtre de sortie plus éloignée, le cas échéant.

Le support peut comporter une semelle définissant la surface de réception précitée, et une partie surélevée sous laquelle le pied peut être engagé, pourvue d'au moins une ouverture sous laquelle au moins un l'un des ongles peut être disposé, la tête optique étant disposée dans l'ouverture ou au-dessus. Dans un exemple de mise en œuvre, la tête optique présente un conduit optique dont la dimension lui permet d'être engagé dans l'ouverture, la tête optique restant par ailleurs en butée sur le support. L'ouverture peut être réniforme, et s'étendre au-dessus de tous les ongles du pied, de telle sorte que l'opérateur peut déplacer le conduit dans l'ouverture au cours du traitement pour traiter plusieurs ongles successivement.

Le support peut comporter au moins un séparateur interdigital disposé de façon à s'engager entre deux doigts lorsque l'ongle à traiter est disposé sous la fenêtre de sortie. Un tel séparateur peut aider à positionner correctement les ongles par rapport à la fenêtre de sortie de la lumière. Le dispositif d'émission de lumière pulsée peut comporter au moins une lampe flash ou un laser.

Le système selon l'invention peut encore comporter, en variante ou en combinaison avec le support, un embout tubulaire présentant une surface intérieure au moins partiellement réfléchissante, l'embout étant fixé à l'une de ses extrémités sur la fenêtre de sortie de la tête optique du dispositif et comportant à son autre extrémité une zone de réception d'au moins un doigt, de telle sorte que l'ongle de ce doigt soit positionné pour recevoir la lumière émise par la tête optique.

Un tel embout permet de positionner la tête optique avec une bonne fiabilité par rapport à l'ongle à traiter. De plus, la forme tubulaire de l'embout et la surface réfléchissante permettent de guider la lumière vers l'ongle à traiter, ce qui peut améliorer le rendement du traitement et protéger de la lumière la personne à traiter.

De préférence, l'embout est en métal, en particulier en aluminium, ce qui lui permet d'être désinfecté facilement entre deux traitements.

L'embout peut être positionné de façon à reposer sur le ou les doigts recevant le traitement. La zone de réception peut comporter au moins un bord incurvé concave venant au contact du ou des doigts, ce qui permet de maintenir l'embout en position tout en protégeant la peau en périphérie du ou des ongles à traiter de la lumière émise.

Le dispositif d'émission peut comporter un panneau de commande permettant de régler les caractéristiques de la lumière émise en fonction de la pathologie et/ou d'au moins une caractéristique de l'ongle, telle que son épaisseur, ce panneau de commande permettant par exemple de sélectionner un préréglage « traitement des ongles » lorsque d'autres applications sont possibles (par exemple épilation). Pour un ongle plus épais, la puissance surfacique pourra être augmentée et/ou le nombre de flashs plus grand.

L'invention a encore pour objet, selon un autre de ses aspects, un système selon la revendication 1, comportant :
- une fenêtre de sortie de la lumière,
- un support pour maintenir une distance prédéfinie entre la fenêtre de sortie et une surface de réception du doigt portant l'ongle à traiter.

Cette distance peut être réglable, le cas échéant, de manière à l'ajuster par exemple à la taille du doigt à traiter (gros orteil ou autre doigt de pied par exemple). Cette distance peut être telle que l'écart entre la fenêtre de sortie et le revêtement soit comprise entre 0 et 6 cm, par exemple entre 2 et 6cm.

Le support peut être en métal ou dans tout autre matériau, par exemple en une matière thermoplastique. Le support peut être réalisé, le cas échéant, dans une matière thermoplastique transparente, ou comporter au moins une partie transparente, permettant par exemple de surveiller le bon positionnement de l'ongle durant le traitement.

Le support peut être réalisé avec une ouverture réniforme, disposée de manière à se superposer à tous les ongles d'un pied, permettant de faire coulisser un conduit optique de la tête de traitement à l'intérieur, ce conduit optique définissant la fenêtre de sortie, pour traiter successivement plus ongles d'un même pied.

L'invention a encore pour objet un système selon la revendication 1 comprenant une gamme de supports ainsi définis, de différentes tailles, permettant à l'opérateur de choisir le support adapté à la taille du pied à traiter.

L'invention a encore pour objet un système selon la revendication 1 comprenant un support destiné à permettre le traitement d'ongles par un dispositif de traitement par émission d'impulsions lumineuses, comportant une partie configurée pour recevoir une tête optique du dispositif, le support ménageant une zone de réception d'au moins un doigt de telle sorte que l'ongle de ce doigt soit positionné pour recevoir la lumière émise par une fenêtre de sortie de la lumière émise par la tête optique, ce positionnement étant de préférence tel que la fenêtre de sortie soit située à une distance comprise entre 1 et 6cm, mieux entre 3 et 6 cm d'une surface sur laquelle le doigt est positionné. Ce support comporte avantageusement au moins un séparateur interdigital.

L'invention a encore pour objet un système selon la revendication 1 comprenant un support destiné à permettre le traitement d'ongles par une tête optique d'un dispositif de traitement par émission d'impulsions lumineuses, notamment pour la mise en œuvre du procédé de traitement selon l'invention, comportant une semelle définissant la surface de réception, et une partie surélevée sous laquelle le pied peut être engagé, pourvue d'au moins une ouverture sous laquelle au moins un l'un des ongles peut être disposé, la tête optique du dispositif étant apte à être disposée dans l'ouverture ou au-dessus de manière à émettre vers les ongles. L'ouverture peut être réniforme, et s'étendre au-dessus de tous les ongles du pied. L'ouverture peut être de dimensions choisies pour guider un conduit optique de la tête optique, dans un mouvement de balayage des différents ongles situés sous l'ouverture.

L'invention a encore pour objet, selon un autre de ses aspects, un système selon la revendication 1 comportant un embout destiné à permettre le traitement d'ongles par une tête optique d'un dispositif de traitement par émission d'impulsions lumineuses, notamment pour la mise en œuvre du procédé de traitement selon l'invention, l'embout comportant :
- un corps tubulaire présentant une surface intérieure réfléchissante, de préférence métallique, notamment en aluminium, et comportant une zone de réception d'au moins un doigt de telle sorte que l'ongle de ce doigt soit positionné pour recevoir la lumière émise par la tête optique, et
- des moyens de fixation du corps tubulaire à la tête optique du dispositif.

Les moyens de fixation peuvent comporter une lanière, par exemple réglable, s'attachant autour de la tête optique, ou tout autre type de fixation adapté, notamment des moyens de fixation magnétiques, par encliquetage, ...

L'invention a encore pour objet, selon un autre de ses aspects, un système selon la revendication 1 comportant un ensemble de patchs à positionner sur des ongles à traiter à l'aide d'un dispositif d'émission d'impulsions de lumière, ces patchs étant de préférence autoadhésifs, prédécoupés avec une forme d'ongle ou de partie d'ongle, et réalisés dans un film absorbant au sens de l'invention, de préférence de couleur sombre, mieux noire, résistant à une température d'au moins 60°C, mieux d'au moins 80°C, mieux d'au moins 100°C ou 150°C, préférentiellement d'au moins 200°C, mieux d'au moins 300°C, de préférence en polyimide ou en PTFE. L'ensemble de patchs peut comporter des patchs en une partie et des patchs en deux parties, notamment en forme de D chacun, comme mentionné plus haut, destinées à se chevaucher sur l'ongle, de différentes tailles. Tous les patchs peuvent être disposés sur un support, de préférence traité antiadhésif, en un nombre suffisant pour traiter l'ensemble des ongles d'une main ou d'un pied. Le film absorbant présente de préférence une réflectivité inférieure à 50% mieux à 20%, et/ou une transmissivité inférieure à 50%, mieux à 20%, vis-à-vis de la lumière émise par le dispositif d'émission d'impulsions lumineuses. Ce film apparaît de préférence opaque à l'œil nu, lorsqu'observé face à la lumière du jour.

La présente divulgation décrit encore un kit de préparation des ongles, comportant un ensemble de patchs tel que défini ci-dessus et un applicateur d'un revêtement réfléchissant, notamment blanc, de préférence dispersable à l'eau.

Un tel kit peut comporter en outre un ensemble d'abrasifs à usage unique, notamment sous forme de cylindres abrasif, agencés pour se monter sur une ponceuse pour abraser l'ongle avant la mise en place du film.

Le kit peut comporter en outre le support selon l'invention, tel que défini plus haut.

Le kit peut encore comporter un aspirateur pour aspirer les poussières émises lors du ponçage de l'ongle.

Le kit peut encore comporter une solution de nettoyage de l'ongle poncé avant l'application du revêtement, notamment une solution aqueuse alcoolique.

### Brève description des dessins

L'invention pourra être mieux comprise à la lecture de la description détaillée qui va suivre, d'exemples de mise en œuvre non limitatifs de celle-ci, et à l'examen du dessin annexé, sur lequel :
[Fig 1] La figure 1 représente de façon schématique un exemple de dispositif de traitement selon l'invention,
[Fig 2] la figure 2 représente en coupe schématique et partielle un ongle muni d'un revêtement selon l'invention,
[Fig 3] la figure 3 illustre le positionnement de l'ongle sous la fenêtre de sortie de la lumière,
[Fig 4] la figure 4 est un schéma en blocs illustrant différentes étapes du procédé de traitement selon l'invention,
[Fig 5] la figure 5 illustre le ponçage de l'ongle,
[Fig 6] la figure 6 illustre la protection de l'environnement cutané de l'ongle,
[Fig 7] la figure 7 illustre la protection du doigt,
[Fig 8] la figure 8 représente un ensemble de masques pour la réalisation du revêtement sur l'ongle,
[Fig 9] la figure 9 illustre la possibilité de superposer plusieurs morceaux de film sur l'ongle,
[Fig 10] la figure 10 représente un exemple de support pouvant équiper la tête de traitement,
[Fig 11] la figure 11 illustre la propagation de la chaleur au sein de l'ongle, à différentes distance de la surface, en fonction du temps,
[Fig 12] la figure 12 représente une variante de support,
[Fig 13] la figure 13 représente un spectre d'émission dit SFL d'un dispositif de traitement par émission de lumière polychromatique pulsée,
[Fig 14] la figure 14 représente un exemple de spectre de transmission de lunettes bleues adaptées à un dispositif de traitement IPL ayant le spectre SFL de la figure 13,
[Fig 15a] [Fig 15b] les figures 15a et 15b représentent différentes vues partielles et schématiques d'un embout pouvant équiper la tête optique du dispositif

### Description détaillée

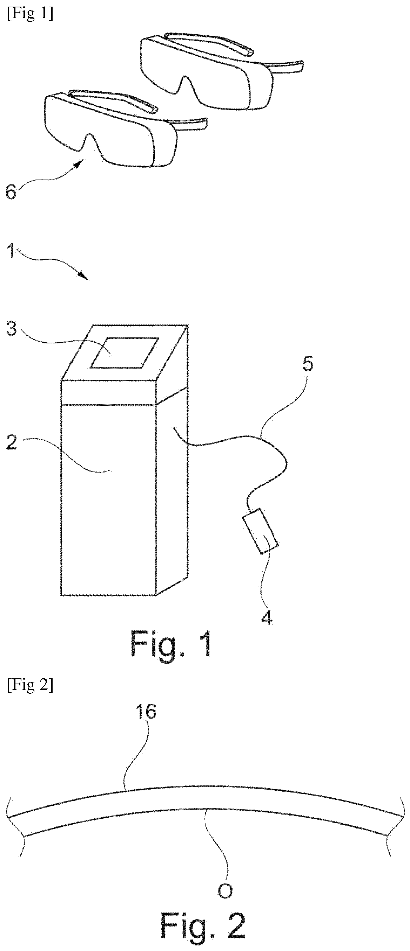

On a représenté à la figure 1 un dispositif 1 de traitement par émission d'impulsions lumineuses, comportant un poste de base 2, muni d'une interface utilisateur 3, et une tête de traitement 4 (encore appelée pièce à main) reliée par un flexible 5 au poste de base.

Le dispositif 1 est par exemple une machine à lumière pulsée de type IPL, la tête de traitement 4 comportant au moins une lampe flash. La machine peut être spécifique au traitement des ongles, ou paramétrable et avoir d'autres applications, par exemple l'épilation.

Des lunettes 6 peuvent être utilisées conjointement avec la machine, conformément à l'enseignement de la demande EP15738647.5 au nom de la demanderesse.

La lumière émise peut avoir le spectre illustré à la figure 13, et les lunettes 6 le facteur de transmission spectral de la figure 14.

Cela permet à l'opérateur utilisant le dispositif de ne pas être ébloui par la lumière émise, et ainsi de pouvoir ainsi contrôler facilement l'application de la lumière pendant la mise en œuvre du traitement.

La tête de traitement 4 peut contenir la ou les lampes flash, comme c'est le cas classiquement pour une machine de type IPL.

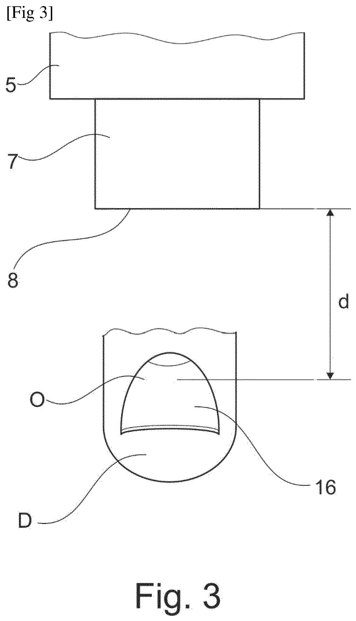

La tête de traitement 4 présente un guide optique 7 dont une extrémité définit une fenêtre 8 de sortie de la lumière, comme illustré à la figure 3. L'ongle O à traiter est placé sous cette fenêtre de sortie 8, avec une distance d entre l'ongle et la fenêtre de sortie par exemple comprise entre 3 et 6cm.

Conformément à l'invention, un revêtement 16 absorbant de manière significative la lumière émise par la fenêtre de sortie 8 recouvre l'ongle. Ce revêtement présente de préférence une réflectivité inférieure à 50% et/ou une transmissivité inférieure à 50%, de manière à s'échauffer de manière importante sous l'effet des flashs de lumière.

Ce revêtement 16 est de préférence constitué par un film d'un matériau plastique opaque résistant à la chaleur, par exemple un polyimide (Kapton) ou du PTFE, de couleur noire. Ce film peut être adhésif ou adhérer à l'ongle électrostatiquement. La transmissivité mesurée au Joule-mètre est par exemple de 13% pour le polyimide et de 6% pour le PTFE, pour des films de couleur noire donnés, convenant à la mise en œuvre de l'invention.

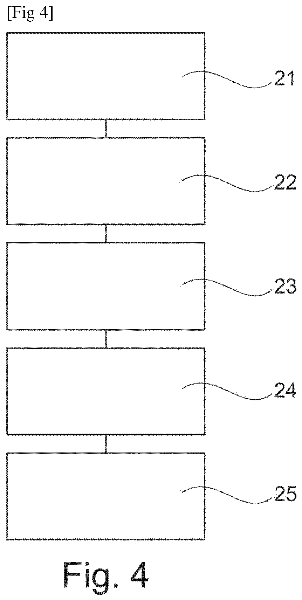

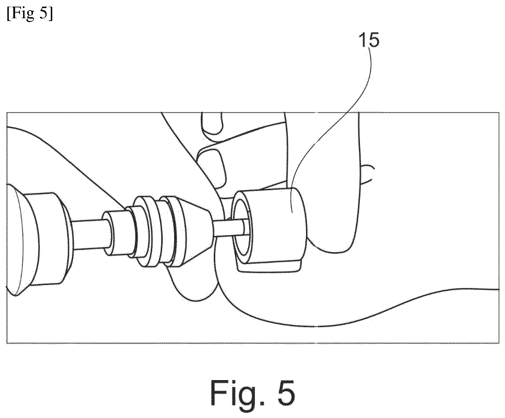

Le procédé de traitement des ongles comporte, comme illustré à la figure 4, une première étape de préparation de l'ongle pour réduire son épaisseur et lisser sa surface. Cette opération est par exemple réalisée à l'aide d'une meule 15 d'un outillage portatif, comme illustré à la figure 5.

Ensuite, à l'étape 22, le revêtement 16 est appliqué sur l'ongle.

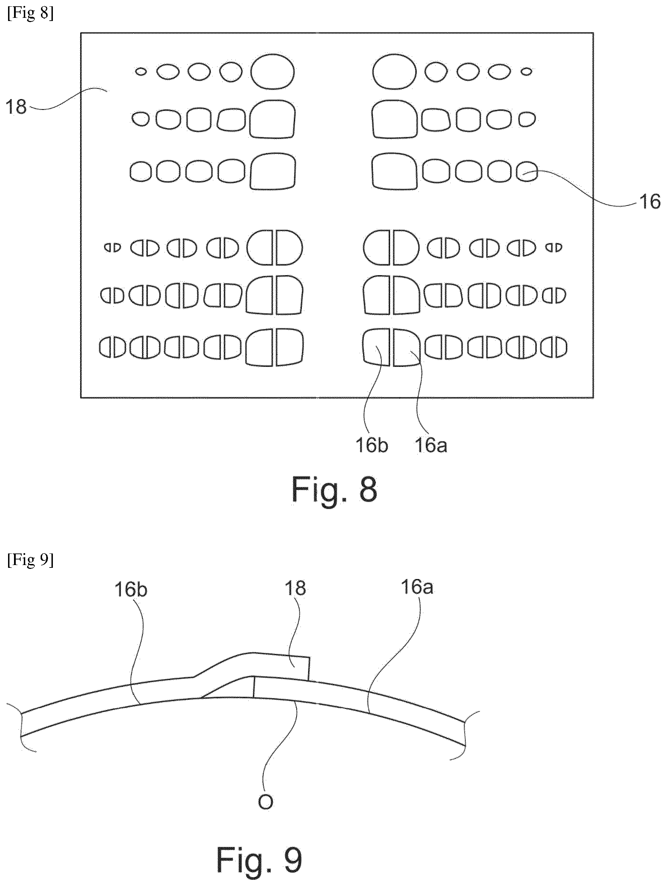

On utilise par exemple un revêtement 16 sous forme de patch prédécoupé à la forme de l'ongle, fourni sur un support 18 antiadhésif, tel qu'illustré à la figure 8.

Le support 18 peut porter des patchs 16 de formats différents, pour s'adapter à la taille de l'ongle. Certains patchs peuvent ne correspondre qu'à une partie de la surface de l'ongle, ce qui est le cas par exemple des patchs 16a et 16b. Il est possible de positionner sur l'ongle deux patchs avec un chevauchement entre eux, comme illustré sur la figure 9. Dans ce cas, on peut commencer par positionner un patch en partant d'un bord de l'ongle, puis l'autre patch est positionné en partant du bord opposé, et collé sur le premier dans la zone de recouvrement.

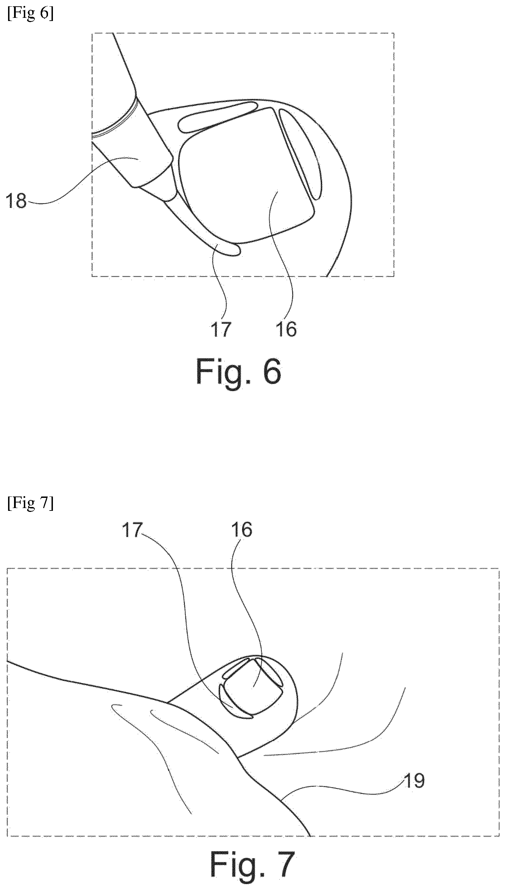

Une fois l'ongle O à traiter muni du revêtement absorbant 16, on peut à l'étape 23 protéger la peau dans sa région au contact de l'ongle autour de celui-ci, en déposant sur la peau un revêtement réfléchissant 17, comme illustré à la figure 6. Ce revêtement 17 est par exemple une encre blanche dispersible à l'eau, appliquée au moyen d'un applicateur 18, par exemple de type pointe applicatrice.

On peut également protéger une partie de la peau du doigt à l'aide d'une feuille blanche 19 d'un matériau souple, comme illustré à la figure 7.

Le traitement par la lumière a lieu à l'étape 24, et consiste à exposer le revêtement à une ou plusieurs rafales de flashs dans l'exemple considéré. A la fin de chaque rafale, l'utilisateur peut ressentir la chaleur amenée sur l'ongle. L'étape 24 peut être renouvelée comme indiqué ci-dessus. Lors du traitement, l'ongle est de préférence maintenu à une distance prédéfinie de la fenêtre de sortie de la lumière de la tête de traitement 4, par exemple à l'aide d'une cale interposée entre le doigt et la pièce à main, ou mieux d'un support dédié, comme détaillé ci-après.

Ensuite, une dernière étape 25 peut consister à retirer le revêtement 16, par pelage par exemple, ainsi que le revêtement blanc, en lavant le doigt.

Cette dernière étape peut avoir lieu sur place ou au domicile de la personne traitée, le cas échéant.

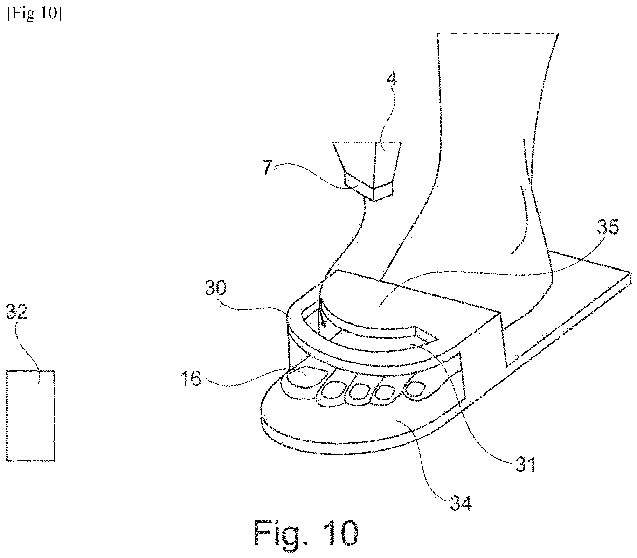

On a représenté à la figure 10 un exemple de support 30 destiné à maintenir la fenêtre de sortie de la lumière à une distance prédéfinie des ongles des pieds. Il présente une semelle 34 définissant une surface de réception du pied, et une partie surélevée 35 reliée à la semelle 34 et sous laquelle le pied est engagé, pourvue d'une ouverture 31 positionnée au-dessus du ou des ongles à traiter, pour le passage de la lumière émise par la tête 4 du dispositif vers le ou les ongles. L'ouverture 31 peut avoir une forme permettant d'y engager le guide optique 7 de la tête, tout en maintenant celle-ci à une distance prédéfinie de la semelle 34. Dans l'exemple illustré, l'ouverture 31 est réniforme, et peut guider le conduit optique de la tête 4 du dispositif dans un mouvement de balayage des ongles des pieds. Par exemple, l'opérateur débute le traitement à proximité d'une extrémité de l'ouverture 31, traite un ongle ou deux, puis déplace le conduit dans le guide pour traiter le ou les ongles suivants.

Un ou plusieurs séparateurs interdigitaux 32 peuvent être utilisés, le cas échéant, en étant disposés entre les orteils.

Le support peut, le cas échéant, être agencé pour se fixer de manière amovible sur la tête de traitement 4, par exemple par une liaison par encliquetage, glissière ou autre, par exemple par vissage.

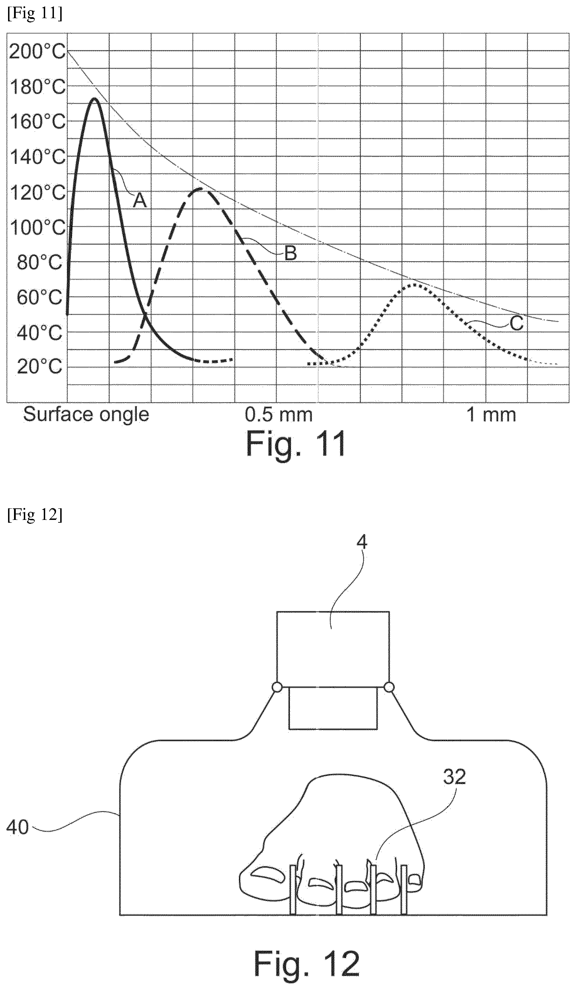

A titre d'exemple, on a représenté à la figure 12 un exemple d'un tel support 40. On voit que ce support 40 peut comporter des séparateurs interdigitaux 32.

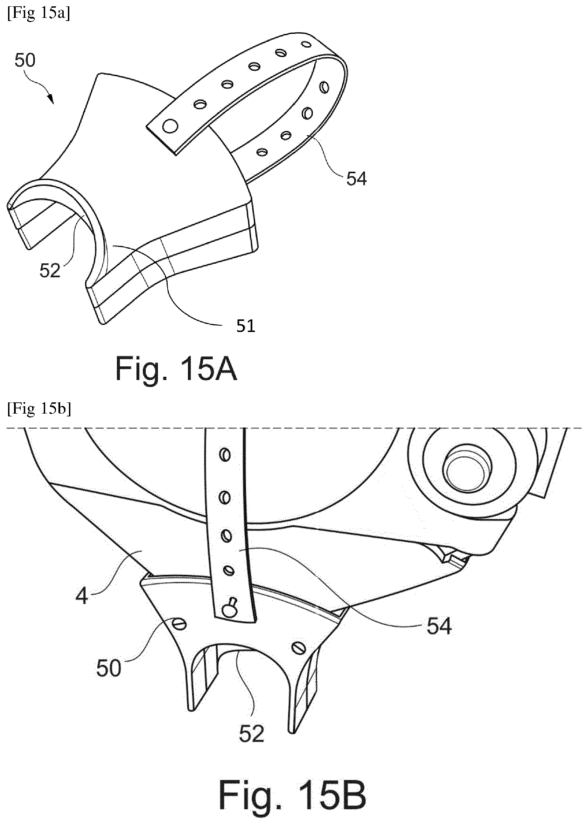

Le dispositif selon l'invention peut encore comporter un embout 50 permettant de guider la lumière vers le ou les ongles à traiter, comme représenté sur la figure 15a et la figure 15b.

L'embout 50 comporte par exemple une lanière 54 lui permettant d'être fixé à la tête optique 4 du dispositif, et un corps tubulaire présentant une zone de réception 51 du ou des doigts dont on souhaite traiter l'ongle. La zone de réception accueille par exemple un ou deux doigts, selon leur taille.

La zone de réception 51 peut comporter un bord incurvé concave 52 qui permet à l'embout de reposer sur le doigt lors du traitement, de telle sorte que l'ongle de ce doigt soit positionné pour recevoir la lumière émise par la tête optique 4. La lumière est guidée de la tête optique à l'ongle à travers le corps tubulaire, qui présente de préférence une surface intérieure réfléchissante, blanche dans l'exemple considéré.

### Exemple

On prépare un ongle affecté par une mycose en le ponçant pour réduire l'épaisseur de l'ongle à 1-2mm ; le ponçage peut s'effectuer avec une ponceuse à ongles, munie d'un cylindre abrasif à usage unique. L'ongle peut être nettoyé à l'alcool. Ensuite, on applique un revêtement constitué d'un film de Kapton auto-adhésif noir sur l'ongle, et l'on protège la peau autour de l'ongle comme décrit plus haut. Le revêtement peut être formé de deux patchs en forme de D, disposés de façon à se chevaucher sur l'ongle sur leurs bords rectilignes.

La machine utilisée dans cet exemple est de type IPL et émet une lumière polychromatique avec le spectre SFL de la figure 13. L'opérateur et la personne traitée portent des lunettes bleues, ces dernières bloquant la quasi-totalité du rayonnement émis par la machine en ayant le facteur de transmission spectral de la figure 14.

On soumet l'ongle à deux rafales de flashs successives à une fréquence de 3Hz. Chaque rafale comporte 14 flashs de 4J/cm² chacun à la sortie de la tête, laquelle se situe à une distance de 3cm de l'ongle. La durée totale de chaque rafale est d'environ 5s. Les deux rafales sont espacées d'environ 2mn. La fluence sur le revêtement est environ 4 fois plus faible qu'à la sortie de la tête, soit environ 1J/cm². La puissance surfacique moyenne sur le revêtement est d'environ 3 W/cm². La température de surface du revêtement, mesurée avec une caméra thermique, dépasse 200°C pendant 0,2s environ, à la fin de la rafale.

L'effet photo-thermique présente trois phases. Il y a d'abord conversion de la lumière en chaleur à la surface du revêtement, puis transfert de celle-ci vers l'intérieur des tissus, et enfin une réaction biologique et cellulaire. Le coefficient d'absorption spectrale de la surface irradiée, associé au spectre d'émission de la source, détermine le pourcentage de photons absorbés et convertis en chaleur. Le transfert de la chaleur s'effectue par conduction thermique.

La chaleur se propage depuis la surface du revêtement selon une vague, tel qu'illustré à la figure 11. Sur cette figure, on a représenté la température en fonction du temps, pour différentes profondeurs à partir de la surface de l'ongle.

On voit qu'à la surface de l'ongle, au contact du revêtement, la température augmente très vite après les flashs pour atteindre près de 180°C, puis décroit en raison de la diffusion de la chaleur dans la matière de l'ongle (courbe A). A mesure que l'on s'éloigne de la surface de l'ongle, la courbe devient plus large et la température maximale décroit (courbe B). A l'interface entre l'ongle et le tissus sous-jacent (courbe C), la température maximale est de 70°C environ, ce qui est insuffisant pour causer une brûlure compte-tenu de la durée pendant laquelle cette température est atteinte, mais suffisant pour détruire les germes responsables la mycose, se localisant dans l'épaisseur de l'ongle et au-dessous de l'ongle.

L'invention, avec la préparation des ongles qu'elle implique, permet d'améliorer considérablement les traitements des ongles par la lumière, en permettant de travailler dans des zones de couple température / temps encore jamais atteintes, notamment des températures plus élevées pour des temps plus courts.

Toutefois, l'invention permet aussi de traiter les ongles avec des températures de surface maximales du revêtement plus basses et des temps plus longs, l'inconvénient de la durée de traitement plus longue étant compensé par la possibilité d'utiliser un dispositif de traitement ayant une puissance lumineuse et une fluence plus faibles.

Bien entendu, l'invention n'est pas limitée à l'utilisation d'une machine IPL à lampe flash et l'on peut également utiliser un laser ou toute autre source adaptée comme source lumineuse.

On peut également utiliser d'autres type de revêtements absorbant la lumière émise lors du traitement, par exemple appliqués sous la forme d'un vernis opaque résistant à la chaleur.

## Revendications

1. Système pour le traitement des ongles, comportant :
- Un dispositif (1) d'émission de lumière pulsée permettant d'exposer au moins un ongle à traiter à au moins une impulsion lumineuse, mieux à une rafale d'impulsions lumineuses,
- au moins un revêtement (16) à appliquer sur le ou les ongles, améliorant la transformation de la lumière en chaleur comparativement à l'ongle nu en absorbant la lumière émise par le dispositif avec une absorptivité supérieure ou égale à 50%, ou un applicateur d'un tel revêtement, et dont la température de surface est apte à dépasser 60°C, mieux 80°C, mieux 100°C, encore mieux 150°C, et plus préférentiellement 200°C, sous l'effet de la lumière émise par le dispositif.

2. Système selon la revendication 1, le revêtement (16) étant de transmissivité inférieure ou égale à 20%, notamment vis-à-vis d'un spectre SFL tel qu'illustré à la figure 13.

3. Système selon l'une des revendications précédentes, le revêtement (16) comportant un film de couleur sombre, notamment noire, résistant à une température d'au moins 200 °C, notamment en PTFE ou en polyimide.

4. Système selon la revendication précédente, le revêtement (16) résistant à une température d'au moins 300°C.

5. Système selon la revendication 3 ou 4, le film (16) étant recouvert sur sa face intérieure à appliquer sur l'ongle d'un adhésif sensible à la pression.

6. Système selon l'une quelconque des revendications 3 à 5, le film (16) étant prédécoupé pour former un patch à la forme d'un ongle ou un ensemble de patchs (16a, 16b) prédécoupés chacun à la forme d'une partie d'ongle, en particulier des patchs en forme de D destinés à se chevaucher sur l'ongle.

7. Système selon l'une quelconque des revendications précédentes, le dispositif d'émission de lumière pulsée comportant au moins une lampe flash.

8. Système selon l'une quelconque des revendications précédentes, comportant un applicateur (18) d'un revêtement réfléchissant (17), notamment blanc, pour former un écran réfléchissant sur la peau à la périphérie de l'ongle.

9. Système selon l'une quelconque des revendications précédentes, le dispositif d'émission de lumière pulsée étant configuré pour émettre une rafale d'impulsions lumineuses à une fréquence comprise entre 0,5 et 20Hz, chaque impulsion présentant une fluence telle, compte-tenu de la distance séparant une fenêtre de sortie du dispositif du revêtement (16) pendant l'utilisation, qu'elle soit comprise entre 0,5 et 5 J/cm² au niveau de la surface du revêtement, mieux 0,5 et 2 J/cm^{2.}

10. Système selon l'une quelconque des revendications précédentes, le dispositif (1) émettant entre les longueurs d'ondes 675nm et 1200nm, le système comportant une paire de lunettes de protection (6) ou un masque de protection de l'opérateur, comportant un filtre absorbant la lumière émise par le dispositif d'émission ayant un facteur de transmission inférieur ou égal à 1% au-delà de 650nm environ, le facteur de transmission de la paire de lunettes étant de préférence tel qu'illustré à la figure 14 pour un spectre d'émission du dispositif tel qu'illustré à la figure 13.

11. Système selon l'une quelconque des revendications précédentes, comportant un support (30 ; 40) pour maintenir une fenêtre de sortie, par laquelle la lumière sort du dispositif, à une distance prédéfinie d'une surface de réception de l'ongle à traiter, notamment une distance (d) comprise entre 0 et 6 cm.

12. Système selon la revendication 11, le support (30) comportant une semelle (34) définissant la surface de réception, et une partie surélevée (35) sous laquelle le pied peut être engagé, pourvue d'au moins une ouverture (31), notamment réniforme, sous laquelle au moins un l'un des ongles peut être disposé, une tête optique étant disposée dans l'ouverture ou au-dessus.

13. Système selon l'une quelconque des revendications précédentes, comportant un embout tubulaire, de préférence métallique, notamment en aluminium, présentant une surface intérieure au moins partiellement réfléchissante, l'embout étant fixé à une de ses extrémités sur la fenêtre de sortie d'une tête optique du dispositif et comportant à son autre extrémité une zone de réception d'au moins un doigt de telle sorte que l'ongle de ce doigt soit positionné pour recevoir la lumière émise par la tête optique.

14. Procédé de préparation d'un ongle en vue de lui faire subir un traitement par émission d'impulsions lumineuses au moyen d'un système selon l'une quelconque des revendications 1 à 13, comportant l'étape consistant à appliquer sur l'ongle un revêtement (16) absorbant la lumière émise durant le traitement, avec une absorptivité supérieure ou égale à 50%, de préférence un revêtement opaque, en particulier de couleur sombre, notamment de couleur noire, et à positionner une fenêtre (8) de sortie d'un dispositif (1) d'émission d'impulsions lumineuses relativement à l'ongle de telle sorte que la lumière émise par le dispositif puisse être dirigée vers l'ongle.

15. Procédé selon la revendication précédente, comportant l'étape consistant à abraser la surface de l'ongle à traiter préalablement à l'application du revêtement absorbant.

## Patentansprüche

1. System zur Behandlung der Nägel, umfassend:
- Eine Vorrichtung (1) zur Emission von gepulstem Licht, die es ermöglicht, mindestens einen zu behandelnden Nagel mindestens einem Lichtimpuls, besser einem Burst aus Lichtimpulsen, auszusetzen,
- mindestens eine auf den oder die Nägel zu applizierende Beschichtung (16), die die Umwandlung von Licht in Wärme verglichen mit dem bloßen Nagel verbessert, indem sie das von der Vorrichtung emittierte Licht mit einer Absorptivität von 50 % oder mehr absorbiert, oder einen Applikator für eine solche Beschichtung, und deren Oberflächentemperatur geeignet ist, 60 °C, besser 80 °C, besser 100 °C, noch besser 150 °C und besonders bevorzugt 200 °C, unter der Wirkung des von der Vorrichtung emittierten Lichts zu überschreiten.

2. System nach Anspruch 1, wobei die Beschichtung (16) eine Transmissivität von 20 % oder weniger aufweist, insbesondere gegenüber einem SFL-Spektrum wie in Fig. 13 dargestellt.

3. System nach einem der vorhergehenden Ansprüche, wobei die Beschichtung (16) eine Folie von dunkler, insbesondere schwarzer, Farbe umfasst, die einer Temperatur von mindestens 200 °C standhält, insbesondere aus PTFE oder Polyimid.

4. System nach dem vorhergehenden Anspruch, wobei die Beschichtung (16) einer Temperatur von mindestens 300 °C standhält.

5. System nach Anspruch 3 oder 4, wobei die Folie (16) auf ihrer auf den Nagel zu applizierenden Innenseite mit einem druckempfindlichen Klebstoff bedeckt ist.

6. System nach einem der Ansprüche 3 bis 5, wobei die Folie (16) vorgeschnitten ist, um einen Aufkleber in Form eines Nagels oder einen Satz von Aufklebern (16a, 16b), die jeweils in Form eines Nagels vorgeschnitten sind, insbesondere Aufkleber in Form eines D, die sich auf dem Nagel überlappen sollen, zu bilden.

7. System nach einem der vorhergehenden Ansprüche, wobei die Vorrichtung zur Emission von gepulstem Licht mindestens eine Blitzleuchte umfasst.

8. System nach einem der vorhergehenden Ansprüche, umfassend einen Applikator (18) für eine reflektierende, insbesondere weiße, Beschichtung (17), um einen reflektierenden Schirm auf der Haut am Rand des Nagels zu bilden.

9. System nach einem der vorhergehenden Ansprüche, wobei die Vorrichtung zur Emission von gepulstem Licht dazu ausgestaltet ist, einen Burst aus Lichtimpulsen mit einer Frequenz zwischen 0,5 und 20 Hz zu emittieren, wobei jeder Impuls eine derartige Fluenz aufweist, dass sie unter Berücksichtigung des Abstands zwischen einem Austrittsfenster der Vorrichtung und der Beschichtung (16) während der Verwendung zwischen 0,5 und 5 J/cm² an der Oberfläche der Beschichtung, besser 0,5 und 2 J/cm², beträgt.

10. System nach einem der vorhergehenden Ansprüche, wobei die Vorrichtung (1) zwischen den Wellenlängen 675 nm und 1200 nm emittiert, wobei das System eine Schutzbrille (6) oder eine Schutzmaske für die Bedienperson umfasst, die einen das von der Emissionsvorrichtung emittierte Licht absorbierenden Filter mit einem Transmissionsfaktor 1 % oder weniger über etwa 650 nm umfasst, wobei der Transmissionsfaktor der Brille vorzugsweise wie in Fig. 14 dargestellt für ein Emissionsspektrum der Vorrichtung wie in Fig. 13 dargestellt ist.

11. System nach einem der vorhergehenden Ansprüche, umfassend eine Halterung (30; 40), um ein Austrittsfensters, durch welches das Licht aus der Vorrichtung austritt, in einem vorgegebenen Abstand von einer Aufnahmefläche des zu behandelnden Nagels, insbesondere einem Abstand (d) zwischen 0 und 6 cm, zu halten.

12. System nach Anspruch 11, wobei die Halterung (30) eine Sohle (34) umfasst, welche die Aufnahmefläche definiert, und einen erhöhten Teil (35), unter den der Fuß eingeführt werden kann und der mit mindestens einer, insbesondere nierenförmigen, Öffnung (31), versehen ist, unter der mindestens einer der Nägel angeordnet werden kann, wobei ein optischer Kopf in der Öffnung oder darüber angeordnet ist.

13. System nach einem der vorhergehenden Ansprüche, umfassend ein rohrförmiges Ansatzstück, vorzugsweise aus Metall, insbesondere aus Aluminium, das eine mindestens teilweise reflektierende Innenoberfläche aufweist, wobei das Ansatzstück an einem seiner Enden an dem Austrittsfenster eines optischen Kopfes der Vorrichtung befestigt ist und an seinem anderen Ende einen Aufnahmebereich für mindestens einen Finger umfasst, so dass der Nagel dieses Fingers so positioniert ist, dass er das von dem optischen Kopf emittierte Licht empfängt.

14. Verfahren zur Vorbereitung eines Nagels im Hinblick darauf, ihn einer Behandlung durch Emission von Lichtimpulsen mittels eines Systems nach einem der Ansprüche 1 bis 13 zu unterziehen, umfassend den Schritt, der darin besteht, auf den Nagel eine Beschichtung (16) zu applizieren, die das während der Behandlung emittierte Licht mit einer Absorptivität von 50 % oder mehr absorbiert, vorzugsweise eine opake Beschichtung, insbesondere von dunkler Farbe, insbesondere von schwarzer Farbe, und ein Austrittsfenster (8) einer Vorrichtung (1) zur Emission von Lichtimpulsen relativ zu dem Nagel so zu positionieren, dass das von der Vorrichtung emittierte Licht zu dem Nagel gerichtet werden kann.

15. Verfahren nach dem vorhergehenden Anspruch, umfassend den Schritt, der darin besteht, die Oberfläche des zu behandelnden Nagels vor dem Applizieren der absorbierenden Beschichtung abzuschleifen.

## Claims

1. System for the treatment of the nails, comprising:
- a pulsed light emission device (1) making it possible to expose at least one nail to be treated to at least one light pulse, better still to a burst of light pulses,
- at least one coating (16) to be applied to the one or more nails, improving the transformation of the light into heat compared with the bare nail by absorbing the light emitted by the device with an absorptivity greater than or equal to 50%, or an applicator of such a coating, of which coating the surface temperature is able to exceed 60°C, better still 80°C, better still 100°C, even better still 150°C, and more preferentially 200°C, under the effect of the light emitted by the device.

2. System according to Claim 1, the coating (16) being of transmissivity less than or equal to 20%, notably with respect to an SFL spectrum as illustrated in Figure 13.

3. System according to one of the preceding claims, the coating (16) comprising a film of dark colour, notably black, resistant to a temperature of at least 200°C, notably made of PTFE or of polyimide.

4. System according to the preceding claim, the coating (16) resisting a temperature of at least 300°C.

5. System according to Claim 3 or 4, the film (16) being covered on its inner face to be applied to the nail with a pressure-sensitive adhesive.

6. System according to any one of Claims 3 to 5, the film (16) being precut to form a patch in the shape of a nail or a set of patches (16a, 16b) that are precut, each in the shape of a part of a nail, in particular D-shaped patches intended to overlap on the nail.

7. System according to any one of the preceding claims, the pulsed light emission device comprising at least one flash lamp.

8. System according to any one of the preceding claims, comprising an applicator (18) of a reflective coating (17), notably white, to form a reflective screen on the skin at the periphery of the nail.

9. System according to any one of the preceding claims, the pulsed light emission device being configured to emit a burst of light pulses at a frequency of between 0.5 and 20 Hz, each pulse having a fluence such that, given the distance separating an output window of the device from the coating (16) during use, it is between 0.5 and 5 J/cm² on the surface of the coating, better still 0.5 and 2 J/cm².

10. System according to any one of the preceding claims, the device (1) emitting between the wavelengths 675 nm and 1200 nm, the system comprising a pair of protective goggles (6) or a protective mask for the operator, comprising a filter absorbing the light emitted by the emission device having a transmission factor less than or equal to 1% above approximately 650 nm, the transmission factor of the pair of goggles preferably being as illustrated in Figure 14 for an emission spectrum of the device as illustrated in Figure 13.

11. System according to any one of the preceding claims, comprising a support (30; 40) for maintaining an output window, through which the light leaves the device, at a predefined distance from a surface for receiving the nail to be treated, notably a distance (d) of between 0 and 6 cm.

12. System according to Claim 11, the support (30) comprising a soleplate (34) defining the receiving surface, and a raised part (35) under which the foot can be fitted, provided with at least one aperture (31), notably reniform, under which at least one of the nails can be disposed, an optical head being disposed in or above the aperture.

13. System according to any one of the preceding claims, comprising a tubular end-fitting, preferably metallic, notably made of aluminium, having an inner surface that is at least partially reflective, the end-fitting being fastened at one of its ends to the output window of an optical head of the device and comprising, at its other end, a zone for receiving at least one digit such that the nail of this digit is positioned to receive the light emitted by the optical head.

14. Method for preparing a nail in order to subject it to a treatment by emission of light pulses by means of a system according to any one of Claims 1 to 13, comprising the step consisting in applying to the nail a coating (16) absorbing the light emitted during the treatment, with an absorptivity greater than or equal to 50%, preferably an opaque coating, in particular of dark colour, notably of black colour, and in positioning an output window (8) of a light pulse emission device (1) relative to the nail such that the light emitted by the device can be directed towards the nail.

15. Method according to the preceding claim, comprising the step consisting in abrading the surface of the nail to be treated prior to the application of the absorbent coating.
